# EUROPEAN PATENT APPLICATION

(11) **EP 4 722 190 A1**
(43) Date of publication of application: **08.04.2026**
(21) Application number: 24306635.4
(22) Date of filing: 04.10.2024
(51) Int. Cl.: C07C 209/60

(54) **SUSTAINABLE SYNTHESIS OF A TERPENYLAMINE IN THE PRESENCE OF AT LEAST ONE ZINC-BASED PRECURSOR**

(71) Applicant: Nantes Université, 44035 Nantes Cedex 1 (FR); Centre National de la Recherche Scientifique, 75016 Paris (FR)
(72) Inventor: RODRIGUEZ-ZUBIRI, Mireia, 44800 Saint Herblain (FR)
(74) Representative: Novagraaf Technologies

(57) **Abstract**

The present invention belongs to the field of aroma, flavours and/or fragrances, and more particularly terpenylamines, which are valuable intermediates in the manufacture of the latter.

The present invention relates to a new process for synthesizing terpenylamines comprising an hydroamination step of at least one terpene with at least one zinc-based precursor and at least one amine, and the use of the synthesized terpenylamines as precursors of a surfactant, a flavour and/or a fragrance.

## Description

### Technical field

The present invention belongs to the field of aroma, flavours and/or fragrances, and more particularly terpenylamines, which are valuable intermediates in the manufacture of the latter.

The present invention relates to a new process for synthesizing terpenylamines in the presence of at least one zinc-based precursor and the use of the synthesized terpenylamine as a precursor of a surfactant and/or a fragrance.

In the description below, references between [ ] refer to the list of references at the end of the examples.

### Technical background

Several processes for the synthesis of terpenylamines, in particular myrcenylamines, have been reported in the prior art.

The hydroamination of myrcene has been studied in the presence of various catalytic systems, mainly based on noble metals such as rhodium **[1]-[2],** palladium **[3]-[18]** and platinum **[19].** Other publications describe the hydroamination of myrcene in the presence of alkali metals **[20]-[28].** There is also an example of the use of alkaline earth metals **[29]** and an example wherein the catalytic system is composed of hexafluoroisopropanol and a Bronsted acid (Tf₂NH 10 mol%) **[30].** A single process for the hydroamination of myrcene has been industrialized (Takasago International Corporation) using diethylamine as the starting amine and in the presence of large quantities of lithium metal as the catalyst **[31].**

While noble metals are scarce and polluting, the lithium-based catalysts use for the only existing industrial hydroamination of myrcene, present several critical drawbacks, including the instability and high cost of the metal, the significant environmental impact of its extraction, and the competition with battery manufacturing for lithium resources.

Overall, the catalytic systems used to obtain myrcenylamines are generally expensive, polluting and/or environmentally unfriendly.

Despite major advances in the search for synthesis processes for myrcenylamines, several challenges remain. This is especially true when it comes to finding synthesis processes applicable to all terpenylamines.

### Detailed description of the invention

Remarkably, the applicant has devised a new process for synthesizing terpenylamines that addresses the aforementioned issues. In fact, the process developed by the applicant can be carried out using cheap, abundant and low-toxicity catalysts on raw materials of plant origin. In addition, it can be implemented without the use of organic solvents, which are polluting volatile compounds.

The present invention relates to a new process for synthesizing terpenylamines in the presence of at least one zinc-based precursor and the use of the synthesized terpenylamines obtained according to the process of the invention, as precursors of surfactants and/or a fragrance.

The process of the invention presents the advantages to be less polluting, more environmentally friendly and more economical than current solutions. Indeed, the use of terpenes and zinc-based precursor in the flavor industry considerably reduces the environmental impact of this fast-growing sector. Terpenes are derived from renewable sources, without competing with the food industry. They represent an alternative to unsaturated hydrocarbons derived from petroleum. On the other hand, zinc-based catalysts are far more abundant, less expensive and less toxic than the noble metals (Pd, Pt, Rh, etc.) commonly used in this type of organic transformation. Zinc-based catalysts enable more sustainable production (more economical and less hazardous) than the lithium-based catalysts used in the only known industrial example to date for the manufacture of myrcenylamines by hydroamination developed by the Japanese company Takasago International Corporation.

The invention thus relates in a first aspect to a process for synthesizing a terpenylamine comprising an hydroamination step of reacting at least one terpene with at least one zinc-based precursor and at least one amine thereby obtaining the terpenylamine.

It is meant by "terpene", a compound with the formula (C₅H₈)ₙ wherein n ≥ 2.

Advantageously, the at least one terpene may be selected from myrcenes, farnesenes, ocimenes, limonenes and mixtures thereof.

Advantageously, the terpene may be a myrcene, preferably 7-methyl-3-methylene-1,6-octadiene, also called β-myrcene.

Advantageously, the terpene may be a farnesene, preferably selected from α-farnesene (3,7,11-Trimethyl-1,3,6,10-dodecatetraene), β-farnesene (7,11-Dimethyl-3-methylene-1,6,10-dodecatriene) and mixtures thereof.

Advantageously, the terpene may be an ocimene, preferably 3,7-dimethyl-1,3,6-octatrien.

Advantageously, the terpene may be a limonene, preferably selected from (S)-(-)-limonene, (-)-*p*-mentha-1,8-diene, (-)-carvene, (*S*)-4-isopropenyl-1-methyl-1-cyclohexene, (*R*)-(+)-limonene, (+)-*p*-mentha-1,8-diene, (+)-carvene, (*R*)-4-isopropenyl-1-methyl-1-cyclohexene, and mixtures thereof.

Advantageously, the terpene may preferably be chosen from the following terpene compounds:

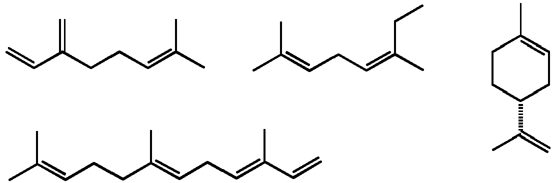

It is meant by "zinc-based precursor", a compound, comprising at least an atom of zinc, participating in a reaction that produces one or more other compounds, for example, this compound may be used to initiate a reaction as an hydroamination.

Advantageously, the at least one zinc-based precursor may be selected from Zn(CF₃SO₃)₂ (CAS 54010-75-2), Zn(ClO₄)₂ × 6 H₂O (CAS 10025-64-6), Zn(OAc)₂ (CAS 557-34-6), Zn(NO₃)₂ × 6 H₂O (CAS 10196-18-6), Zn(CN)₂ (CAS 557-21-1), Zn(BF₄)₂ × nH₂O (CAS 27860-83-9), ZnF₂ (CAS 7783-49-5), ZnCl₂ (CAS 7646-85-7), ZnBr₂ (CAS 7699-45-8), ZnI₂ (CAS 10139-47-6), ZnO (CAS 1314-13-2), ZnS (CAS 1314-98-3), ZnSe (CAS 1315-09-9), Zn(O*ⁱ*Pr)₂ (CAS 13282-39-8), Zn(SO₄) × H₂O (CAS 7446-19-7), Zn[N(SO₂CF₃)₂]₂ (CAS 168106-25-0), Zn(acac)₂ × nH₂O (CAS 108503-47-5), Zn(acacCF₃)₂ × 2H₂O (CAS 16743-33-2), preferably from Zn(CF₃SO₃)₂, Zn(ClO₄)₂ × 6 H₂O, Zn(BF₄)₂ × H₂O.

It is meant by "amine", a compound and/or a functional group that contains a basic nitrogen atom with a lone pair. Usually, amines are derivatives of ammonia, wherein one or more hydrogen atoms have been replaced by a substituent such as an alkyl or aryl group.

Advantageously, the at least one amine is selected from: aromatic amines, for example aniline and derivatives of anilines such as *N*-methylaniline, *N*-substituted anilines and anilines containing at least one substituent on the aromatic ring in *ortho, meta* or *para* positions; aliphatic amines for example morpholine or diethylamine; preferably from aromatic, primary or secondary, amines and from aliphatic, primary or secondary, amines, and more preferably from aromatic secondary amines. Preferably, the at least one amine is chosen from *N*-methylaniline (CAS 100-61-8), *N*-methyl-4-(trifluoromethyl)aniline (CAS 22864-65-9), *N*-methyl-4-fluoroaniline (CAS 459-59-6), *N*-methyl-4-nitroaniline (CAS 100-15-2), *N*-methyl-*p*-toluidine (CAS 623-08-5), 4-(trifluoromethyl)aniline (CAS 455-14-1), 4-nitroaniline (CAS 100-01-6), 4-fluoroaniline (CAS 371-40-4), 4-chloroaniline (CAS 106-47-8), 4-bromoaniline (CAS 106-40-1), *p*-anisidine (CAS 104-94-9), 4-isopropylaniline (CAS 99-88-7), 4-ethylaniline (CAS 589-16-2), *p*-toluidine (CAS 106-49-0), 2,6-dimethylaniline (CAS 87-62-7), 3,5-bis(trifluoromethyl)aniline (CAS 328-74-5), 4-(methylamino)pyridine (CAS 1121-58-0), aniline (CAS 62-53-3), morpholine (CAS 110-91-8), diethylamine (CAS 109-89-7), and mixtures thereof.

It is meant by "hydroamination", a step of addition of an N-H bond of an amine across a carbon-carbon multiple bond of an alkene, alkyne, diene, or allene.

Advantageously, the hydroamination step of the process according to the invention may be carried out in the presence of a ligand.

It is meant herein by "ligand", an organic molecule containing one or more nitrogen, oxygen or phosphorous heteroatoms available to bind the zinc metal centre. The ligand may be chosen from pyridine derivatives, salen derivatives, *N-*heterocyclic carbene derivatives or phosphane derivatives.

It is meant by "pyridine derivatives", compounds comprising at least one pyridine moiety.

It is meant by "salen derivatives", compounds comprising at least one primarily bis-Schiff base.

It is meant by "*N*-heterocyclic carbene derivatives", compounds comprising at least one cyclic carbene species with two neighboring nitrogen atoms.

It is meant by "phosphane derivatives", compounds comprising at least one three-valent phosphorus atom surrounded by three substituents of formula P*ₙ*R_{*n*+2} (R being chosen from alkyl, aryl, alcoxy and aryloxy group).

Advantageously, the ligand may be chosen from the following ligands: Xantphos (CAS 161265-03-8), triphenylphosphine (CAS 603-35-0), rac-BINAP (CAS 98327-87-8), DPPF (CAS 12150-46-8), SPhos (CAS 657408-07-6), tri-*tert-*butylphosphine (CAS 13716-12-6), RuPhos (CAS 787618-22-8), tri(*o-*tolyl)phosphine (CAS 6163-58-2), BrettPhos (CAS 1070663-78-3), di(1-adamantyl)-*n*-butylphosphine (CAS 321921-71-5), *t*BuXPhos (CAS 564483-19-8), tris(dimethylamino)phosphine (CAS 1608-26-0), (2-biphenyl)di-*tert*-butylphosphine (CAS 224311-51-7), DavePhos (CAS 213697-53-1), tributylphosphine (CAS 998-40-3), 1,3-bis(diphenylphosphino)propane (CAS 6737-42-4), *t*BuBrettPhos (CAS 1160861-53-9), 1,4-bis(diphenylphosphino)butane (CAS 7688-25-7), trimethylphosphine (CAS 594-09-2), ethylenebis(diphenylphosphine) (CAS 1663-45-2), tributylphosphine (CAS 998-40-3), CyJohnPhos (CAS 247940-06-3), tris(diethylamino)phosphine (CAS 2283-11-6), 1,2-bis(diphenylphosphino)benzene (CAS 13991-08-7), tris(trimethylsilyl)phosphine (CAS 15573-38-3), tri(2-furyl)phosphine (CAS 5518-52-5), 1,2-bis(dicyclohexylphosphino)ethane (CAS 23743-26-2), tris(4-methoxyphenyl)phosphine (CAS 855-38-9), tri-*n*-butylphosphine (CAS 998-40-3), (oxydi-2,1-phenylene)-bis(diphenylphosphine) (CAS 166330-10-5), tris(4-fluorophenyl)phosphine (CAS 18437-78-0), JackiePhos (CAS 1160861-60-8), 1,1'-bis(di-*tert*-butylphosphino)ferrocene (CAS 84680-95-5), bis(diphenylphosphino)methane (CAS 2071-20-7), tris(4-trifluoromethylphenyl)phosphine (CAS 13406-29-6), *N*-XantPhos (CAS 261733-18-0), diphenyl-2-pyridylphosphine (CAS 37943-90-1), tris(1-pyrrolidinyl)phosphine (CAS 5666-12-6), ethylenebis(diphenylphosphine) (CAS 1663-45-2), 1,5-bis(diphenylphosphino)pentane (CAS 27721-02-4), bis(2-diphenylphosphinoethyl)phenylphosphine (CAS 23582-02-7), 2,6-bis(di-*tert-*butylphosphinomethyl)pyridine (CAS 338800-13-8), tripropylphosphine (CAS 2234-97-1), 2-(2-(diphenylphosphino)ethyl)pyridine (CAS 10150-27-3), 1,1'-bis(phenylphosphinidene)ferrocene (CAS 72954-06-4), TyrannoPhos (CAS 2097604-70-9), EvanPhos (CAS 2223634-83-9), isopropyldiphenylphosphine (CAS 6372-40-3), 1,3-bis(2,4,6-trimethylphenyl)imidazol-2-ylidene (IMes), 1,3-bis(2,4,6-trimethylphenyl)-4,5-dihydroimidazol-2-ylidene (SIMes, CAS 173035-11-5), 1,3-bis(2,6-Diisopropylphenyl)imidazol-2-ylidene (IPr), 4,5-dichloro-1,3-bis(2,6-Diisopropylphenyl)imidazol-2-ylidene, 1,3-bis(2,6-diisopropylphenyl)-4,5-dihydroimidazol-2-ylidene (SIPr), 1,3-bis(2,6-dibenzhydryl-4-methylphenyl)imidazol-2-ylidene (IPr*), 1,3-bis(cyclohexyl)imidazol-2-ylidene (ICy), 1,3-bis(cyclohexyl)-4,5-dihydroimidazol-2-ylidene (SICy), 1,3-bis(trimethyl)imidazol-2-ylidene (IMe), 1,3-bis(trimethyl)-4,5-dimethylimidazol-2-ylidene (Me₂IMe), 1,3-bis(tert-butyl)imidazol-2-ylidene (I^{t}Bu), 1,3-bis(adamantyl)imidazol-2-ylidene (lAd), 2-(2-Pyridyl)indole (CAS 13228-40-5), 2,6-Bis(4-phenyl-2-oxazolin-2-yl)pyridine, 2-(2-Pyridyl)benzoxazole (CAS 32959-62-9), 1,2-Ethanediamine, *N,N,N',N'*-tetrakis(2-pyridinylmethyl) (CAS 16858-02-9), trans-2,6-Diisopropyl-*N*-(2-pyridylmethylene)aniline (CAS 908294-68-8), *N,N*-bis(2-pyridinylmethyl)-2-Pyridinemethanamine (CAS 16858-01-8), 2-(2-pyridinyl)-1H-Indole (CAS 13228-40-5), *N,N*'-Bis(2-pyridylmethyl)-1,2-ethylenediamine, 1,1'-(2,6-Pyridinediyl)bis(3-methylimidazol-2-ylidene), 2,2'-bipyridine, 2,2' ;6',2"-terpyridine, 1,10-phenanthroline, 2-pyridinemethanol, 8-hydroxyquinoline, *N*-benzyl-1-(pyridin-2-yl)methanimine, 2-((benzylimino)methyl)phenol, *N,N'*-(ethane-1,2-diyl)bis(1-phenylmethanimine) and *N,N*'-(cyclohexane-1,2-diyl)-bis(1-phenylmethanimine).

Advantageously, the ligand may preferably be chosen from the following ligands:

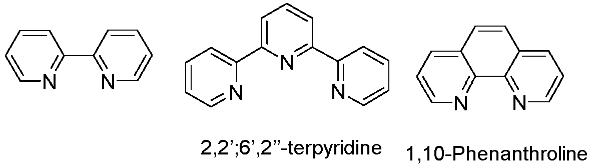

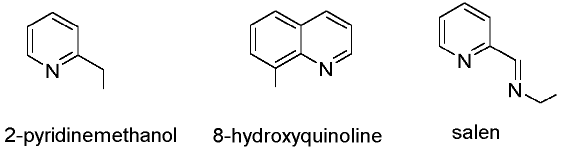

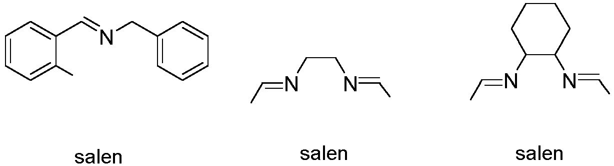

Advantageously, the step of hydroamination may be carried out with a molar ratio terpene:amine comprised from 1:1 to 1:10, preferably 1:10.

Advantageously, the step of hydroamination may be carried out with a molar ratio terpene:zinc-based precursor comprised from 1:0.2 to 1 :0.001, preferably 1:0.1.

Advantageously, the step of hydroamination is carried out with a molar ratio terpene:ligand comprised from 1:0.4 to 1:0.001, preferably 1:0.1.

Advantageously, the step of hydroamination may be carried out for a duration from 5 minutes to 72 hours, preferably from 5 minutes to 48 hours, more preferably from 5 hours to 48 hours, or for a duration of 48 hours.

Advantageously, the step of hydroamination may be carried out at a temperature from 25 to 125 °C, preferably from 100 to 110 °C.

Advantageously, the step of hydroamination is carried out under neat conditions. The step of hydroamination may optionally be carried out in a solvent. The solvent may be water or an organic solvent. The organic solvent may be chosen from 1,1,1,3,3,3-hexafluoroisopropanol, 2,2,2-trifluoroethanol, dichloromethane, acetonitrile, methanol, acetone, hexanes, 1,4-dioxane, *n*-propanol, *n*-butanol, isopropanol, DMF, DMSO, tetrahydrofuran and toluene.

Advantageously, the step of hydroamination is carried out in the presence of an internal reference.

It is meant by "internal reference", a compound useful for the quantification of the reaction products of the step of hydroamination, said internal reference being inert in the conditions of the reaction and having a retention time different from the other species involved. The internal reference may be chosen from alkanes having a boiling point higher than the temperature at which the step of hydroamination is carried on. The internal reference may for example be n-dodecane. The skill person may choose an internal reference adapted to the embodiment of the invention that they intend to carry on.

The invention relates in another aspect to the use of a terpenylamine as obtained according to the process of the present invention, as a precursor of a surfactant, a flavour and/or a fragrance.

Advantageously, the flavour and/or fragrance may be selected from nerol, linalool, geraniol, (+)-citronellal, (-)-menthol, (+)-citronellol, myrcenol, hydroxycitronellal.

Advantageously, the terpenylamine may be synthetized from a myrcene (β-myrcene) and the flavour and/or fragrance may be selected from nerol, linalool, geraniol, (+)-citronellal, (-)-menthol, (+)-citronellol, myrcenol, hydroxycitronellal.

The following represents an example of a general reaction of hydroamination of β-myrcene according to the process of the invention, with the most described isomers:

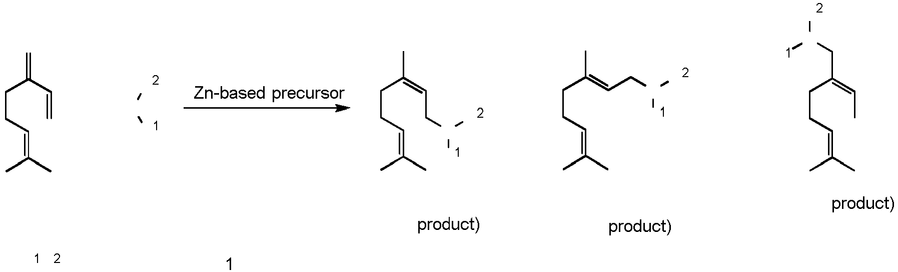

Advantageously, the terpenylamine may be synthetized from a farnesene (β-farnesene) and the surfactant may be selected from laurics-based surfactants.

The following represents an example of a general reaction of hydroamination of farnesene according to the process of the invention with the most described isomers:

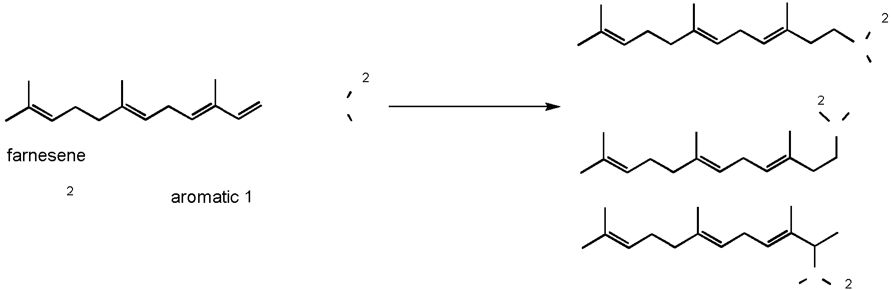

Advantageously, the terpenylamine may be synthetized from an ocimene (cis-β-ocimene) and the flavour and/or fragrance may be selected from nerol, linalool, geraniol, (+)-citronellal, (-)-menthol, (+)-citronellol, myrcenol, hydroxycitronellal.

### Brief description of the figures:

Figure 1 represents the yields of the myrcenyl-*N*-methylaniline afforded in the hydroamination of β-myrcene with representative examples of zinc-based precursors.
Figure 2 represents the yields of the myrcenyl-*N*-methylaniline afforded in the hydroamination of β-myrcene with Zn(CF₃SO₃)₂ and representative examples of pyridine-derivative organic ligands.
Figure 3 represents the yields of the myrcenyl-*N*-methylaniline afforded in the hydroamination of β-myrcene with Zn(CF₃SO₃)₂ and representative examples of salen-derivative organic ligands.
Figure 4 represents the yields of the myrcenyl-*N*-methylaniline afforded in the hydroamination of β-myrcene with Zn(CF₃SO₃)₂ and representative organic solvents.

The invention will be further illustrated by the following examples.

### Examples

Representative results are shown in the following tables and graphs for the example of the hydroamination of β-myrcene with the *N*-methylaniline.

Preliminary blank tests in the absence of zinc-based precursors have confirmed that the hydroamination of β-myrcene cannot proceed without at least one zinc-based catalysts (Table 1).

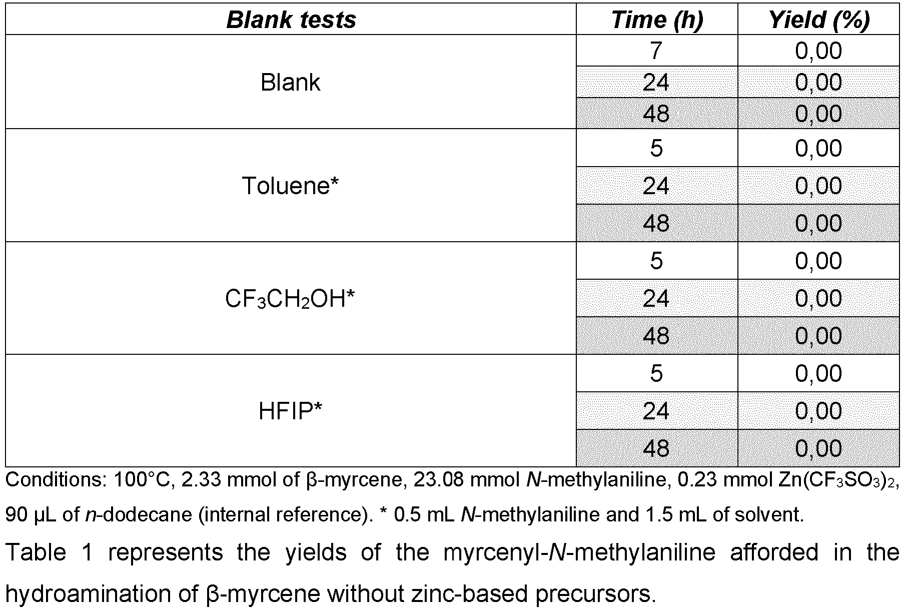

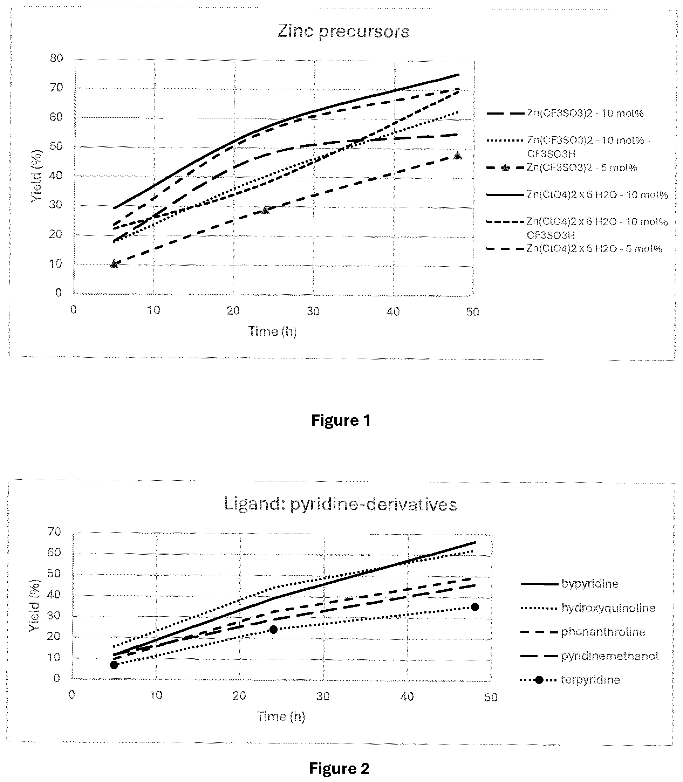

The presence of at least one zinc-based precursor allows the hydroamination of β-myrcene to produce the myrcenyl-*N*-methylaniline, more precisely, the "tail" anti-markovnikov isomer useful as precursor of fragrances and flavours. The desired product was obtained in good to excellent yields (Table 2 and Figure 1).

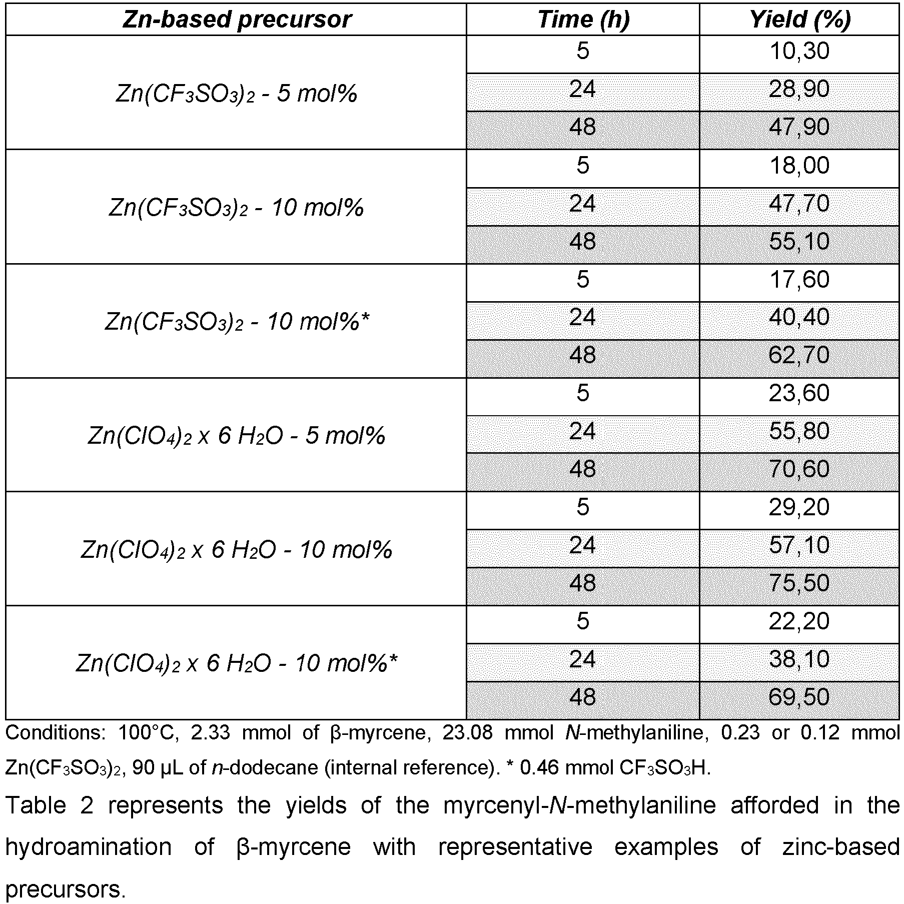

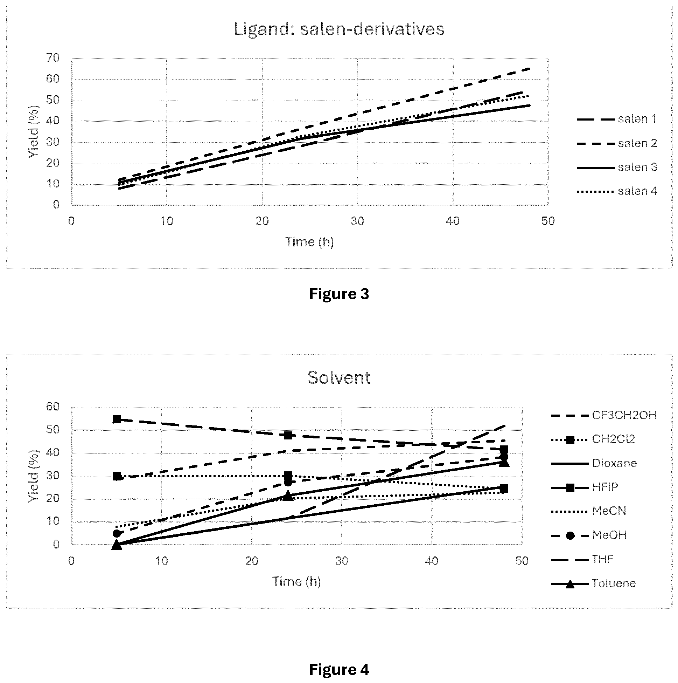

The performance of the hydroamination of terpenes (for instance β-myrcene) in the presence of at least one zinc-based precursor (for instance Zn(CF₃SO₃)₂) and one organic ligand containing a nitrogen, an oxygen and or a phosphorous atom, was evaluated. Some representative results (Table 3, Figure 2 and Figure 3) have revealed that the hydroamination, *e.g*. the hydroamination of β-myrcene, successfully proceeded in the presence of the named ligands, paving the way to new applications and the development of new catalytic processes.

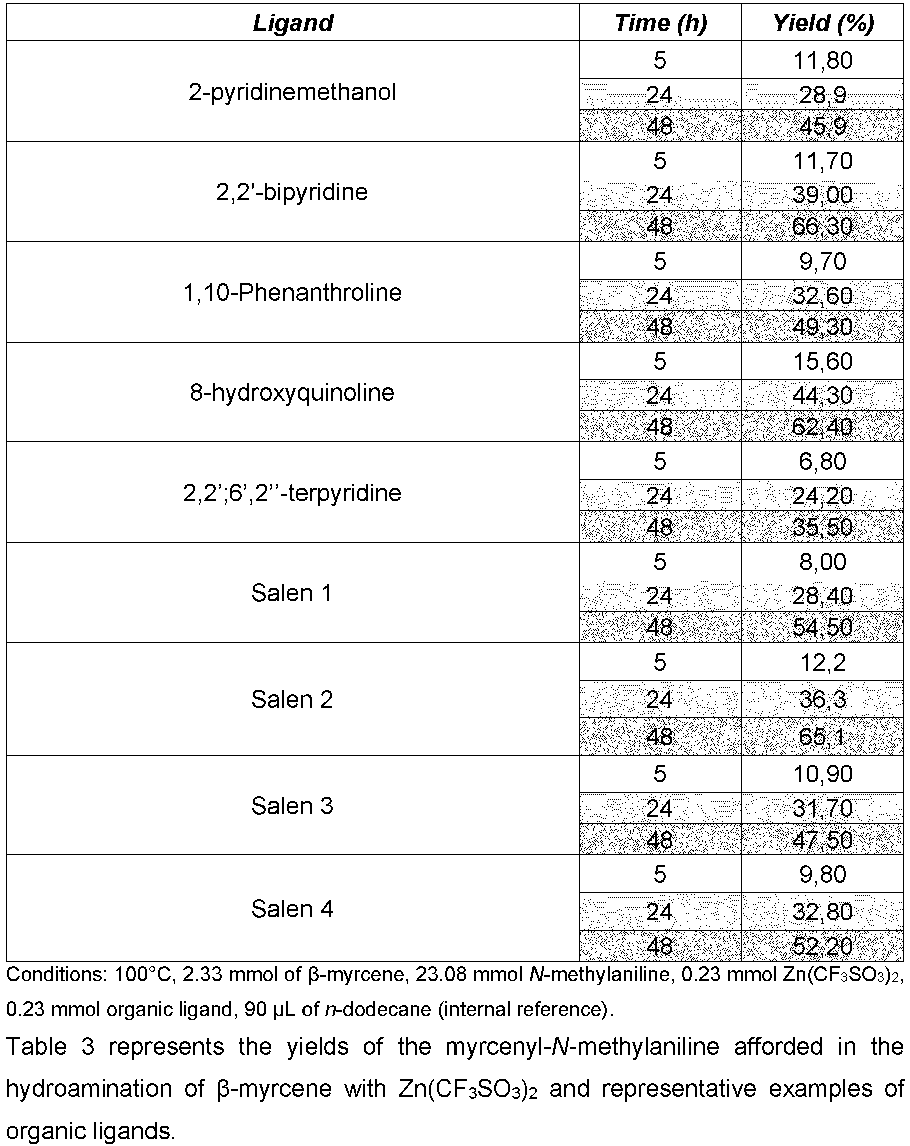

It is important to note that the hydroamination reaction according to the invention affords better yields of the desired product under neat conditions than in the presence of at least one organic solvent (Table 4, Figure 4). Nevertheless, THF gives interesting yields. Surprisingly, HFIP gives excellent yields in only 5h.

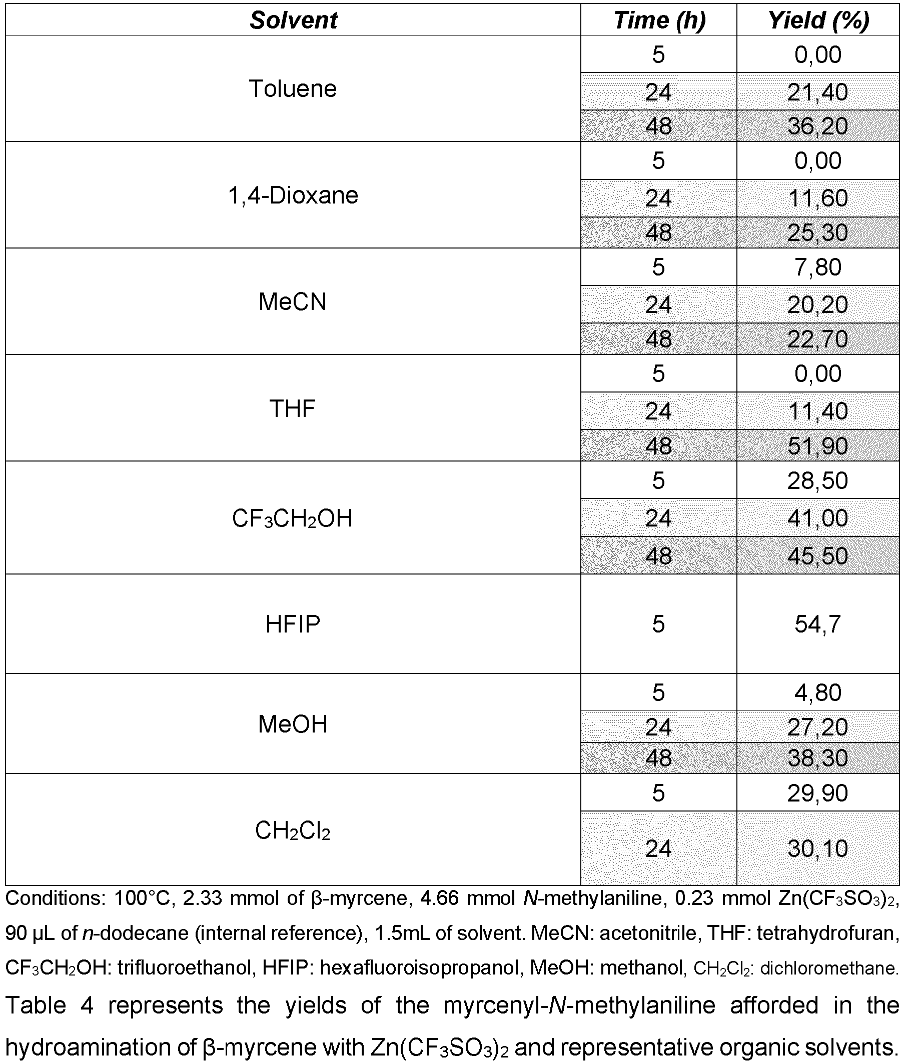

### EXPERIMENTAL: Synthesis of terpenylamines according to the process according to the invention

General information: All commercially available chemicals were used as received unless otherwise noted. High-field ¹H and ¹³C NMR spectra were recorded at 300 or 400 and 75 or 100 MHz, respectively. ¹H and ¹³C NMR spectra were referenced to the residual signal of the internal deuterated solvent (CDCl₃) at 7.26 and 77.16 ppm, respectively, and coupling constants were measured in Hertz.

The GC analyses were performed on a GC-FID Agilent 7820A chromatograph equipped with a 30 m HP5 capillary column. The GC-MS analyses were recorded on a TRACE GC Ultra (ThermoScientific) apparatus equipped with a 30 m TR-5MS silica capillary column and a DSQII quadrupole analyser (ThermoScientific). High resolution mass spectrometry (HRMS) was recorded on a microTOF spectrometer equipped with orthogonal electrospray interface (ESI). Analytical thin-layer chromatography (TLC) was carried out on silica gel 60 F254 plates and visualized with a UV lamp at 254 nm or stained with a basic potassium permanganate solution. Flash column chromatography was performed using silica gel 60 (40-63 µm).

### Experimental procedures:

### General procedure for the preparation of terpenylamines:

The appropriate amount of catalyst corresponding to 1-20 mol% of zinc-based precursor was transferred into a test tube under inert gas atmosphere in order to weight the right amount of catalysts without adventitious water due to the hygroscopic nature of the zinc precursors.

The appropriated amount of amine (2.33-23.08 mmol), *n*-dodecane (90 µL) and the desired terpene (2.33 mmol) were then added without preserving the inert gas atmosphere. The mixture was stirred in an oil bath thermostated at the desired temperature (25-125 °C) for the desired reaction time (5 to 48 hours). Liquid aliquots (10 µL) of the reaction mixture were analyzed by gas chromatography (GC) and gas chromatography-mass spectrometry (GC-MS) analysis after 5 min, 1h, 2h, 5h, 24h and 48h of reaction time. Quantification of the reaction products was possible with the internal standard (*n*-dodecane). Identification of the reaction products was possible using GC-MS analysis. At the end, the reaction mixture was cooled and the solvent was removed under reduced pressure. The crude was purified by flash chromatography on silica and evaporated to dryness. The expected products (myrcenyl amine isomers) were identified by GC-MS, High-Resolution Mass Spectroscopy (HRMS) and ¹H and ¹³C NMR techniques.

### General procedure for the preparation of terpenylamines in the presence of organic solvents:

The appropriate amount of catalyst corresponding to 1-20 mol% of zinc-based precursor was transferred into a test tube under inert gas atmosphere in order to weight the right amount of catalysts without adventitious water due to the hygroscopic nature of the zinc precursors. Solvent (1.5 mL) was added without preserving the inert gas atmosphere and the mixture was sonicated for 10 min.

The appropriated amount of amine (2.33-23.08 mmol), *n*-dodecane (90 µL) and the desired terpene (2.33 mmol) were then added. The mixture was stirred in an oil bath thermostated at the desired temperature (25-125 °C) for the desired reaction time (5 to 48 hours). Liquid aliquots (10 µL) of the reaction mixture were analyzed by gas chromatography (GC) and gas chromatography-mass spectrometry (GC-MS) analysis after 5 min, 1h, 2h, 5h, 24h and 48h of reaction time. Quantification of the reaction products was possible with the internal standard (*n*-dodecane). Identification of the reaction products was possible using GC-MS analysis. At the end, the reaction mixture was cooled, and the solvent was removed under reduced pressure. The crude was purified by flash chromatography on silica and evaporated to dryness. The expected products (myrcenyl amine isomers) were identified by GC-MS, High-Resolution Mass Spectroscopy (HRMS) and ¹H and ¹³C NMR techniques.

Nature of solvents: Water, toluene, 1,4-dioxane, acetonitrile, tetrahydrofuran, dichloromethane, alcohols (some examples are methanol, 1,1,1,3,3,3-hexafluoroisopropanol, 2,2,2-trifluoroethanol).

### General procedure for the preparation of terpenylamines in the presence of additives as Brönsted acids and or organic ligands.

The appropriate amount of catalyst corresponding to 1-20 mol% of zinc-based precursor was transferred into a test tube under inert gas atmosphere in order to weight the right amount of catalysts without adventitious water due to the hygroscopic nature of the zinc precursors.

The appropriated amount of amine (2.33-23.08 mmol), additive (10-20 mol% of a Brönsted acid and/or 1-20 mol% of an organic ligand), *n*-dodecane (90 µL) and the desired terpene (2.33 mmol) were then added without preserving the inert gas atmosphere. The mixture was stirred in an oil bath thermostated at the desired temperature (25-125 °C) for the desired reaction time (5 to 48 hours). Liquid aliquots (10 µL) of the reaction mixture were analyzed by gas chromatography (GC) and gas chromatography-mass spectrometry (GC-MS) analysis after 5 min, 1h, 2h, 5h, 24h and 48h of reaction time. Quantification of the reaction products was possible with the internal standard (*n*-dodecane). Identification of the reaction products was possible using GC-MS analysis. At the end, the reaction mixture was cooled and the solvent was removed under reduced pressure. The crude was purified by flash chromatography on silica and evaporated to dryness. The expected products (myrcenyl amine isomers) were identified by GC-MS, High-Resolution Mass Spectroscopy (HRMS) and ¹H and ¹³C NMR techniques.

### Nature of the additives:

Bronsted acids: Sulfonic acids (some examples are CF₃SO₃H, ClSO₃H, *p*-toluene sulfonic acid, benzene sulfonic acid, CH₃SO₃H, camphor sulfonic acid), boronic acids (some examples are HBF₄), carboxylic acids (some examples are CF₃CO₂H), phosphonic acids and hydrogen halides.

### Conclusion

In summary, herein is reported a process for synthesizing a terpenylamine in the presence of at least one zinc-based precursor, which presents the advantage to be less polluting, more environmentally friendly and more economical than current solutions.

### List of references

**[1]** G. Mignani, D. Morel (Rhône-Poulenc, France), FR2569403,1986.
**[2]** A. Behr, L. Johnen, ChemSusChem 2009, 2,1072-1095.
**[3]** X.-H. Yang, A. Lu, V. M. Dong, J. Am. Chem. Soc. 2017,139,14049-14052.
**[4]** A. Behr, L. Johnen, 41. Jahrestreffen Deutscher Katalytiker, Weimar, 2008.
**[5]** A. Behr, L. Johnen, Dream Reactions-Synthesis and Processes for Sustainable Chemistry, Aachen, 2008.
**[6]** A. Behr, L. Johnen, ChemSusChem 2009, 2,1072-1095.
**[7]** A. Behr, L. Johnen, N. Rentmeister, Adv. Synth. Catal. 2010, 352, 2062-2072
**[8]** T. Fàrber, R. Schulz, O. Riechert, T. Zeiner, A. Gorak, G. Sadowski, A. Behr, Chem. Eng. Process 2015, 98, 22-31
**[9]** T. Fârber, O. Riechert, T. Zeiner, G. Sadowski, A. Behr, A. J. Vorholt, Chem. Res. Des. 2016,112, 263-273
**[10]** D. Vogelsang, J. M. Dreimann, D. Wenzel, L. Peeva, J. da Silva Burgal, A. G. Livingston, A. Behr, A. J. Vorholt, Ind. Eng. Chem. Res. 2017, 56, 13634-13641
**[11]** A. Perrier, M. Ferreire, J. N. H. Reek, J. I. van der Vlugt, Catal. Sci. Technol. 2013, 3,1375.
**[12]** D. Banerjee, K. Junge, M. Bel 1er, Angew. Chem. Int. Ed. 2014, 53,1630 - 1635.
**[13]** W.-S. Jiang, D.-W. Ji, W.-S. Zhang, G. Zhang, X.-T. Min, Y.-C. Hu, X.-L. Jiang, Q.-A. Chen, Angew. Chem. Int. Ed. 2021, 60, 8321-8328.
**[14]** A. Y. Jiu, H. S. Slocumb, C. S. Yeung, X.-H. Yang, V. M. Dong, Angew. Chem. Int. Ed. 2021, 60,19660 -19664.
**[15]** A. Behr, L. Johnen, A. J. Vorholt, ChemCatChem 2010, 2, 1271 - 1277.
**[16]** T. A. FaBbach, R. Kirchmann, A. Behr, A. J. Vorholt, Green Chem., 2017,19, 5243-5249.
**[17]** M. Terhorst, A. Kampwerth, A. Marschand, D. Vogt, A. J. Vorholt, T. Seidensticker, Catal. Sci. Technol., 2020,10,1827.
**[18]** A. Kampwerth, M. Terhorst, N. Kampling, D. Vogt, T. Seidensticker, Green Chem., 2023, 25, 6345.
**[19]** R. Schulz, L. Sengen, K. Giebelmann, S. Hunold, T. Zeiner, Chem. Ing. Tech. 2018, 90 (7), 947-955.
**[20]** K. Takabe, T. Katagiri, J. Tanaka, T. Fujita, S. Watanabe, K. Suga, Org. Synth. 1989, 67, 44.
**[21]** T. Fujita, K. Suga, S. Watanabe, Aust. J. Chem. 1974, 27, 531.
**[22]** K. Sugahara, T. Fujita, S. Watanabe, H. Hashimoto, J. Chem. Technol. Biotechnol. 1987, 37, 95.
**[23]** G. Hata, M. Tanaka (Toray Industries, Japan), JP51070707,1976.
**[24]** A. J. Chalk, S. A. Magennis, Ann. N. Y. Acad. Sci. 1980, 333, 286.
**[25]** Z. Wang, J. Zang, Y. Wu, Jingxi Huagong Zhongjianti 2005, 35, 42.
**[26]** J. Peng, H. Zhanao, C. Zhaogang, C. Wenchang, Z. Jinbao, Z. Zhangzheng (Shanghai Wanxiang Daily Chemicals Co., Ltd.), CN101602651A, 2009.
**[27]** P. E. Alekseevna, K. D. Vladimirovich (INEOS RAS), RU2533831, 2014.
**[28]** S. Heng, L. Haiyan, W. Jiagao, S. Chuanwei (Synergetica Changzhou Ltd), CN 102850227, 2013.
**[29]** C. Brinkmann, A. G. M. Barrett, M. S. Hill, P. A. Procopiou, J. Am. Chem.Soc. 2012,134, 2193-220.
**[30]** S. Pradhan, S. Das, G. Kumar, I. Chatterjee, Org. Lett. 2022, 24, 2452-2456.
**[31]** M. Emura, H. Matsuda, Chem Biodivers 2014, 11(11), 1688-1699.

## Claims

1. A process for synthesizing a terpenylamine comprising an hydroamination step of reacting at least one terpene with at least one zinc-based precursor and at least one amine thereby obtaining a terpenylamine.

2. The process according to claim 1, wherein the terpene is a terpene selected from myrcenes, farnesenes, ocimenes, limonenes and mixtures thereof.

3. The process according to claim 1 or 2, wherein the terpene is a myrcene, preferably β-myrcene.

4. The process according to claim 1 or 2, wherein the terpene is a fanesene, preferably selected from α-farnesene, β-farnesene and mixtures thereof.

5. The process according to claim 1 or 2, wherein the terpene is an ocimene, preferably 3,7-dimethyl-1,3,6-octatrien.

6. The process according to claim 1 or 2, wherein the terpene is a limonene, preferably selected from (*S*)-(-)-limonene, (-)-*p*-mentha-1,8-diene, (-)-carvene, (*S*)-4-isopropenyl-1-methyl-1-cyclohexene, (*R*)-(+)-limonene, (+)-*p-*mentha-1,8-diene, (+)-carvene, (*R*)-4-isopropenyl-1-methyl-1-cyclohexene, and mixtures thereof.

7. The process according to any one of the preceding claims, wherein the at least one zinc-based precursor is selected from Zn(CF₃SO₃)₂, Zn(ClO₄)₂ × 6 H₂O, Zn(OAc)₂, Zn(NO₃)₂ × 6 H₂O, Zn(CN)₂, Zn(BF₄)₂ × nH₂O, ZnF₂, ZnCl₂, ZnBr₂, ZnI₂, ZnO, ZnS, ZnSe, Zn(O*ⁱ*pr)₂, Zn(SO₄) × H₂O, Zn[N(SO₂CF₃)₂]₂, Zn(acac)₂ × nH₂O, Zn(acacCF₃)₂ × 2H₂O.

8. The process according to any one of the preceding claims, wherein the step of hydroamination is carried out in the presence of a ligand.

9. The process according to any one of the preceding claims, wherein the at least one amine is selected from aromatic amines and aliphatic amines, preferably *N*-methylaniline.

10. The process according to any one of the preceding claims, wherein the step of hydroamination is carried out with a molar ratio terpene:amine comprised from 1:1 to 1:10.

11. The process according to any one of the preceding claims, wherein the step of hydroamination may be carried out with a molar ratio terpene:zinc-based precursor comprised from 1:0.2 to 1:0.001.

12. The process according to any one of claims 8 to 13, wherein the step of hydroamination is carried out with a molar ratio terpene:ligand comprised from comprised from 1:0.4 to 1:0.001.

13. The process according to any one of the preceding claims, wherein the step of hydroamination is carried out for a duration from 5 minutes to 72 hours.

14. The process according to any one of the preceding claims, wherein the step of hydroamination is carried out at a temperature from 25 to 125 °C.

15. The use of the terpenylamine obtained according to the process of any of the preceding claims as a precursor of a surfactant, a flavour and/or a fragrance.
